# EUROPEAN PATENT SPECIFICATION

(11) **EP 2 825 535 B1**
(45) Date of publication and mention of the grant of the patent: **27.04.2016**
(21) Application number: 13716178.2
(22) Date of filing: 14.03.2013
(51) Int. Cl.: C07D 451/10

(54) **A METHOD OF PREPARING THE SCOPINE ESTER OF DI-(2-THIENYL)GLYCOLIC ACID, AN INTERMEDIATE IN THE SYNTHESIS OF TIOTROPIUM BROMIDE, AND ITS NEW FORM**
VERFAHREN ZUR VORBEREITUNG DER SCOPINESTER VON DI-(2-THIENYL) GLYKOLSÄURE, EIN ZWISCHENPRODUKT IN DER SYNTHESE VON TIOTROPIUMBROMID UND SEINE NEUE FORM
PROCÉDÉ DE PRÉPARATION DE L'ESTER DE SCOPINE DE L'ACIDE DI-(2- THIÉNYL)GLYCOLIQUE, UN INTERMÉDIAIRE DANS LA SYNTHÈSE DU BROMURE DE TIOTROPIUM, ET SA NOUVELLE FORME

(30) Priority: 16.03.2012 CZ 20120190
(43) Date of publication of application: 21.01.2015
(73) Proprietor: Zentiva, K.S., 102 37 Praha 10 (CZ)
(72) Inventor: CERNA, Igor, 067 16 Vyrava (SK); HAJICEK Josef, 250 81 Nehvizdy (CZ)
(74) Representative: Jirotkova, Ivana
(86) International application number: PCT/CZ2013/000041
(87) International publication number: WO 2013/135219

(56) References cited:
- EP-A1- 0 418 716
- WO-A1-2011/015884
- WO-A1-2011/123077

## Description

### Technical Field

The invention relates to a new preparation method of the scopine ester of di-(2-thienyl)glycolic acid (hereinafter the scopine ester only) of formula **I.** The scopine ester of formula **I** is an important intermediate in the synthesis of tiotropium bromide, a substance with the chemical name (1R,2R,4S,5S,7S)-7-(2-hydroxy-2,2-di(thiophen-2-yl)acetoxy)-9,9-dimethyl-3-oxa-9-azatricyclo[3.3.1.0^{2,4}]nonan-9-ium bromide, of formula **II.**

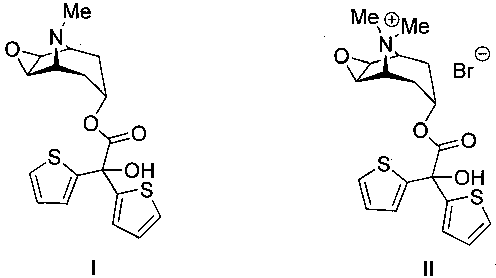

### Background Art

Tiotropium bromide of formula **II,** first described in patent EP 0 418 716, is a selective, competitive, reversible antagonist of cholinergic receptors with a long-term effect. Unlike the structurally similar ipratropium it selectively blocks the muscarinic receptors M1 and M3, while it blocks receptors M2 for a short time. It has significant bronchodilating effects. It is especially used to treat the chronic obstructive pulmonary disease (COPD) and asthma. The therapeutic dosage of the active substance is in micrograms, in the form of powder, which is administrated bymeans of an inhalation device.

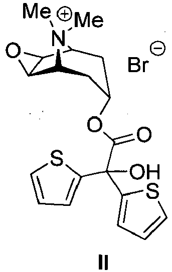

With regard to low therapeutic doses of the active substance, the pharmaceutical production of the substance and the process of its preparation is subject to high requirements not only concerning the yield of the process and its economical and environmental aspects, but also requirements for high purity of the resulting active substance.

A preparation process of tiotropium bromide was first published in patent EP 0 418 716 and is illustrated in Scheme 1.

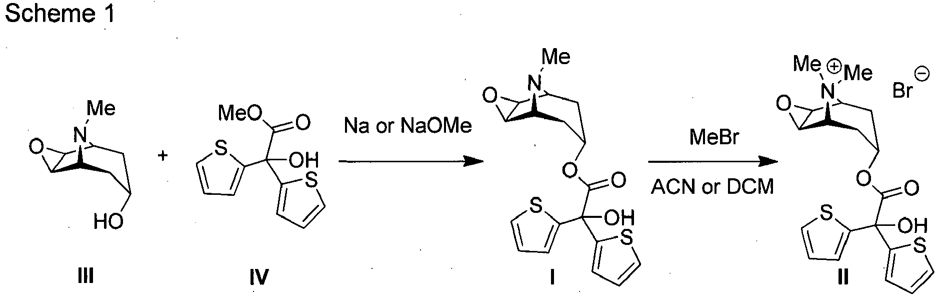

The first step is transesterification of methyl-di(2-thienyl)glycolate of formula **IV** with scopine of formula **III** in a strongly basic environment. The reaction is carried out in an organic solvent (toluene, xylene, heptane) or preferably in a melt. According to the patent metallic sodium, sodium hydride, sodium methoxide or sodium ethoxide is used as the strong base. The described method uses strongly basic conditions; the amount of the base varies from substoichiometric (0.1 - 0.36 equivalents per mole of scopine) if metallic sodium is used, to stoichiometric amounts (1.0 equivalent per mole of scopine) if sodium methoxide is used as the base. The temperature of the reaction should not exceed 95°C; the reaction is carried out at a reduced pressure (50 kPa to 4 kPa). The reaction mixture is processed by acidification, the acidic phase is subsequently alkalized to prepare a free base, which is extracted with dichloromethane. The product is crystallized from acetonitrile and this way a crystalline product is obtained, here referred to as form A, with the melting point of 148 to 150°C. The yields of the synthesis of the scopine ester **I** vary in the range from 45% to 70%.

As can be seen, the above mentioned examples describing preparation of the scopine ester use harsh conditions that could hardly be used to synthesize the product in the industrial scale.

It is especially the combination of using metallic sodium and carrying out the reaction in a melt at high temperatures and reduced pressure that poses high risks when employed in a larger scale in pharmaceutical production.

The second step is quaternization. The reaction of the scopine ester of formula I with methyl bromide is carried out in acetonitrile or in a solvent mixture of dichloromethane and acetonitrile. The subsequent re-crystallization was performed in a not precisely specified mixture of methanol and acetone and a white crystalline product with the melting point of 217 to 218°C was obtained. The patent examples do not mention any yield of quaternization with methyl bromide.

An alternative method of synthesis of tiotropium bromide is described in patent US 6 506 900 B1 and is illustrated in Scheme 2.

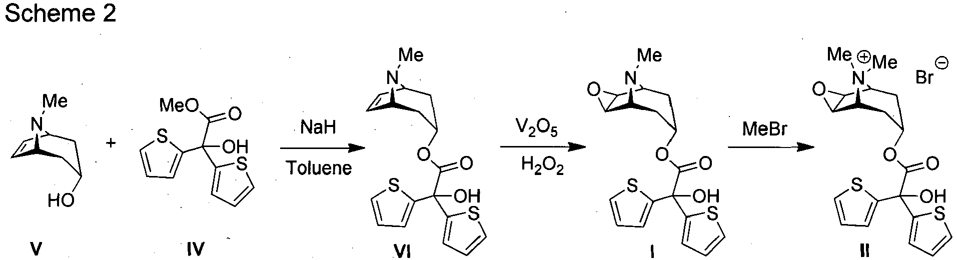

It starts with tropenol of formula **V,** which is used in a reaction with methyl-di(2-thienyl)glycolate of formula **IV** in the presence of at least the stoichiometric amount of sodium hydride (preferably 1.5-2.0 equivalents per mole of tropenol) at a temperature of 60 to 75°C and a pressure of 25 to 30 kPa. The second step is oxidation of the tropenol ester of formula **VI** to the scopine ester of formula **I** with the use of vanadium dioxide and hydrogen peroxide, and the subsequent quaternization provides tiotropium bromide of formula **II** in the total yield of 71%. Even though the selected procedure is referred to as an improvement of the method described in the basic patent, it uses a higher than stoichiometric amount of the strong base for the transesterification and the use of tropenol as the starting compound adds an epoxidation step to the synthesis.

Another patent US 6 747 154 B2 mentions a procedure wherein scopine methobromide of formula **VII** is used instead of scopine in the reaction with di(2-thienyl)glycolic acid of formula **VIII** or its derivatives. The reaction proceeds in a dipolar aprotic solvent (*N*-methylpyrrolidinone, dimethylacetamide) under basic conditions in the presence of the lithium salt of imidazole (Scheme 3). The patent does not mention any experimental details, including yields, of using this reaction for synthesis of tiotropium bromide.

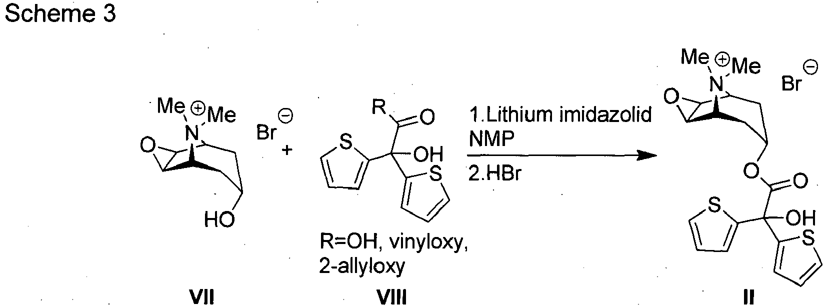

Application US2006/0047120 presents an improvement of the above mentioned method from the point of view of control of impurities, using protection of the free hydroxyl group of the sodium salt of di(2-thienyl)glycolic acid of formula **IX** with the trimethylsilyl group (Scheme 4). Although this is an *in situ* production of the protected derivative of formula **X,** it increases complexity of the synthesis. The yields of the sequence executed this way vary in the range of 34-85% of the resulting tiotropium bromide of formula **II.**

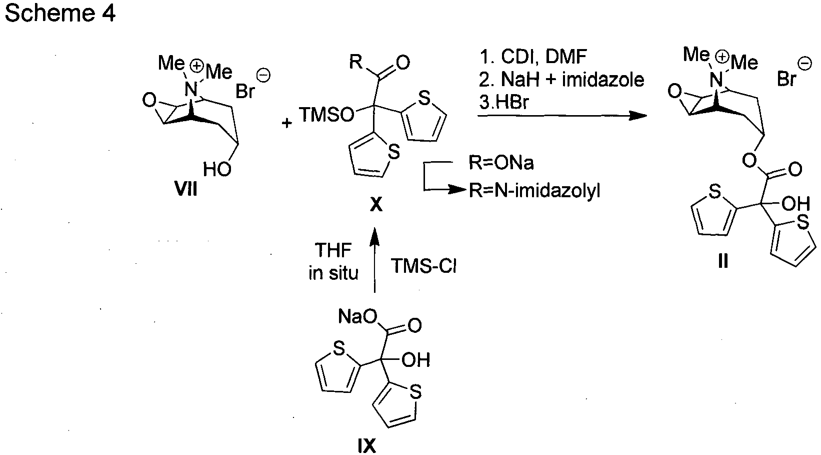

Patent application WO 2008/008376 describes the transesterification procedure using an excess (2.5 equivalents per mole of scopine hydrobromide, or 1.5 equivalents per mole of scopine) of an anhydrous inorganic base (K₂CO₃) in dimethylformamide at the temperature of 65°C and reduced pressure of 7 to 10 kPa (Scheme 5). The subsequent quaternization with methyl bromide was performed in acetonitrile at the room temperature. The total yield of tiotropium bromide was 42 to 61 %.

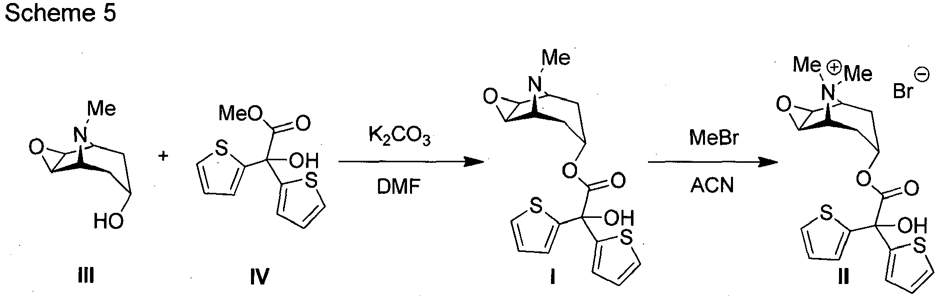

In patent application WO 2009/087419 scopine of formula **III** is used for the transesterification of methyl-di(2-thienyl)glycolate of formula **IV** in the hydrochloride salt form (Scheme 6). The reaction is pertformed in DMF at 60°C in basic conditions in the presence of an organic amine (1 to 3 equivalents of 1,8-diazabicyclo[5.4.0]undec-7-ene; DBU), preferably in combination with another base (1 to 2 equivalents of NaH per mole of scopine hydrochloride). The subsequent quaternization with methyl bromide can also be carried out without isolation of the scopine ester of formula **II,** but such a procedure provides the resulting product in an unsuitable purity and low yield. The total yield is 41 to 50%.

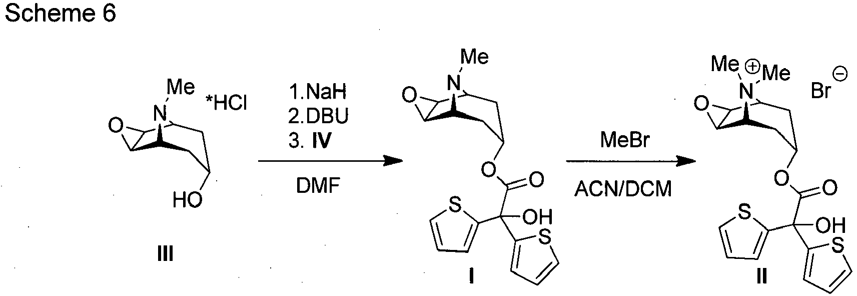

Nearly all the above mentioned methods use as the key step (trans)esterification of di-(2-thienyl) glycolic acid or its derivatives with scopine of formula **III,** or with scopine methobromide of formula **VII** in the presence of a base. However, these conditions lead to the formation of scopoline and its related impurities to a higher or lower extent (Scheme 8).

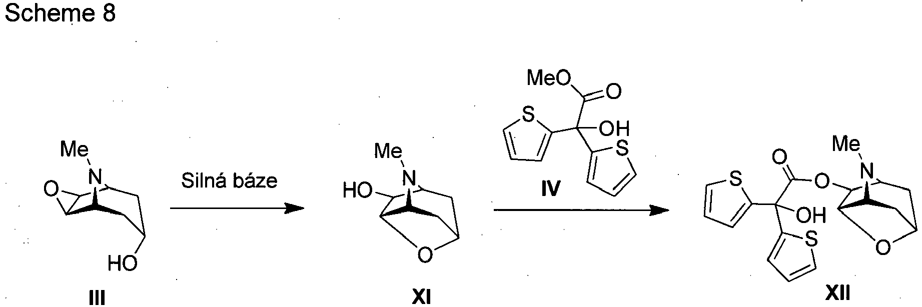

Thus, a new approach to synthesis of tiotropium bromide of formula **II** that would circumvent the above mentioned critical point of (trans)esterification would considerably improve control over scopoline impurities of formula **XI** and **XII,** contributing to preparation of tiotropium bromide of formula **II** in a better purity.

### Disclosure of Invention

The invention provides a new efficient method of preparation of the scopine ester of formula I 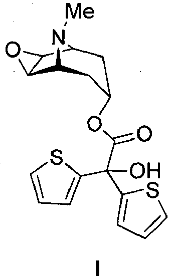 which consists of the following steps:
a) reaction of scopine of formula III with derivatives of oxalic acid of formula **XIII** 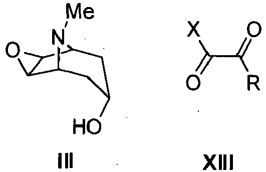 wherein X means F, Cl, Br, or I, and R is X or *O*-*tert*-butyl, *O*-methyl, N-pyrrolidinyl, *N*-morpholinyl or *N*-imidazolyl, in the presence of a weak base and a catalyst, in an inert organic solvent, producing a derivative of formula **XIV** 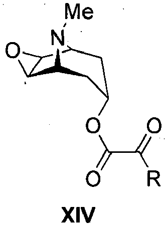
b) reaction of the derivative of formula **XIV** with at least 2 equivalents of 2-thienylmagnesium bromide of formula **XV** 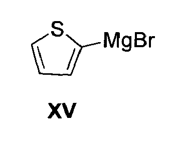
c) isolating the resulting scopine ester of formula **I** and crystallizing it from a crystallization solvent.

The new method of synthesis of tiotropium bromide is shown in Scheme 7.

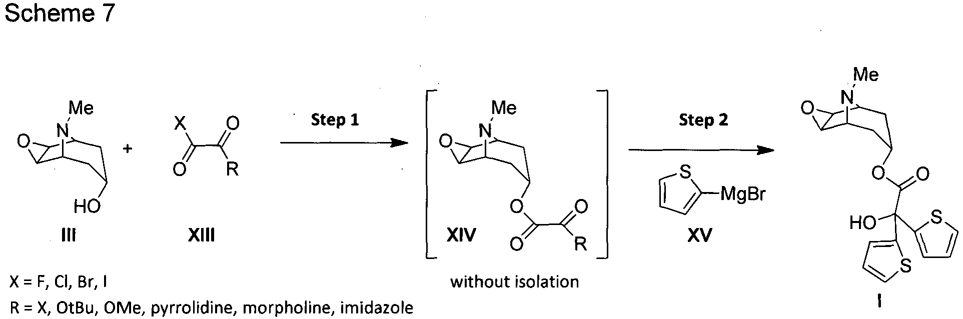

The new preparation method consists of two steps, which are preferably carried out without isolation of the intermediate of formula **XIV.**

The first step consists in a reaction of scopine of formula **III** with a derivative of oxalic acid of formula **XIII,** wherein:
X is selected from the group of the halogens fluorine, chlorine, bromine or iodine; chlorine being selected in a preferable embodiment,
R is selected from the group of halogens, or also *O*-*tert*-butyl, *O*-methyl, *N-*pyrrolidinyl, *N*-morpholinyl and *N*-imidazolyl, the substituent R being preferably selected from the group of halogens, and chlorine being selected in a preferable embodiment.

For the reaction, 1-3 equivalents of the oxalic acid derivative of formula **XIII** are used per mole of scopine of formula **III;** in a preferable embodiment, 1-1.05 equivalents of the compound of formula **XIII** are used.

The reaction is performed by controlled addition of a solution of a mixture of scopine of formula **III** with a weak base to a pre-cooled solution of the oxalic acid derivative of formula **XIII** in the presence of a catalyst.

The reaction is carried out in an inert organic solvent, such as ethers, or cyclic ethers, especially tetrahydrofuran.

The addition of scopine of formula **III** is carried out slowly and to a mixture of the oxalic acid derivative of formula **XIII,** which has been cooled down to a temperature of -10 °C to 15°C, preferably to a mixture which has been cooled down to 0°C.

The weak base is selected from the group of tertiary amines; preferably triethylamine is used as the base in an amount of 1-1.5 equivalents per mole of scopine of formula **III;** more preferably 1 equivalent is used.

Dimethylaminopyridine in an amount of 5 to 10 mol%, preferably 5 mol%, is used as the catalyst.

After the addition the reaction mixture is reacted at 0°C for 2h.

The second step is started by cooling the reaction mixture to the temperature of -30°C, followed by controlled addition of 2-thienylmagnesium bromide to the cooled reaction mixture.

For the reaction, 2-3.5 equivalents of 2-thienylmagnesium bromide are used per mole of scopine of formula **III,** preferably 3 equivalents of 2-thienylmagnesium bromide are used.

After the addition the reaction mixture is reacted at -30°C for 3 h.

After completion of the reaction of the starting compounds the produced scopine ester of formula **I** is converted to a salt by pouring of the reaction mixture into a cooled aqueous solution of an inorganic acid, hydrochloric acid being used in a preferable embodiment. By shaking of the acidic phase with a suitable organic solvent most organic impurities are removed from the salt of the scopine ester of formula **I.** Suitable organic solvents include dichloromethane or toluene.

The aqueous layer containing the salt of the scopine ester of formula **I** is alkalized with a suitable base to pH in the range of 8 to 9; an aqueous solution of sodium carbonate being preferably used as the base. The product is subsequently extracted with a suitable organic solvent, dichloromethane being conveniently used as the solvent.

This method provides the product, i.e. scopine ester of formula **I,** with the purity of 80.0 to 85.0% (determined by UPLC), preferably in a purity higher than 80% (determined by UPLC).

Crystallization of the crude product obtained this way is carried out in a suitable solvent, using acetonitrile or toluene, preferably acetonitirile. Crystallization is carried out from a solution of the scopine ester of formula **I** in acetonitrile at -5°C to -40°C, preferably at -10 to -30°C. The crystalline product is isolated and dried at the normal pressure or *in vacuo* at temperatures in the range from the room temperature to 50°C. This way the new crystalline form B of the scopine ester of formula **I** can also be prepared, which differs from the known form A published in patent EP 0 418 716 by its characteristic melting point in the range of 115 to 120°C.

Form B of the scopine ester of formula **I** further differs from the known form A by manifesting the following characteristic reflections in the X-ray powder record, measured by means of CuKα radiation: 10.03; 12.00; 14.03; 16.53; 20.10; 26.24 +/0.2° 2Th.

Form B of the scopine ester of formula **I** exhibits further characteristic reflections in the X-ray powder record: 8.24; 13.09; 18.13; 18.94; 23.18; 24.27; 25.03; 26.92; 33.43 +/- 0.2° 2Th.

The product of formula **I** is isolated in the purity of 97.0 to 99.0%, conveniently in a higher purity than 97.5% (determined by UPLC).

The second step of synthesis of tiotropium bromide of formula **II** is quaternization of the scopine ester of formula **I** with methyl bromide, which is carried out analogously to the procedure described in the basic patent EP 0 418 714.

### Brief Description of Drawings

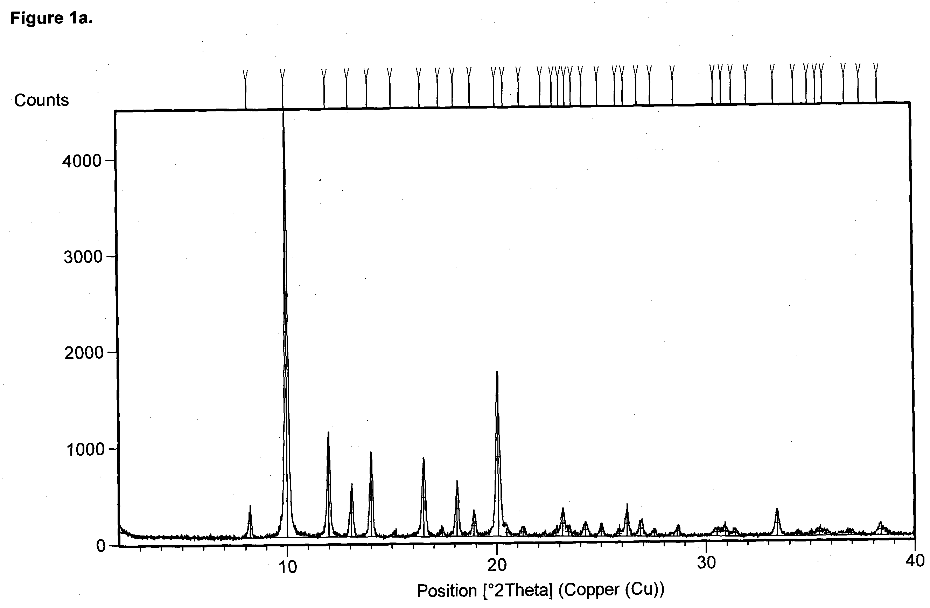

**Figure 1a****.** X-ray powder record of the scopine ester of formula **I**, form B

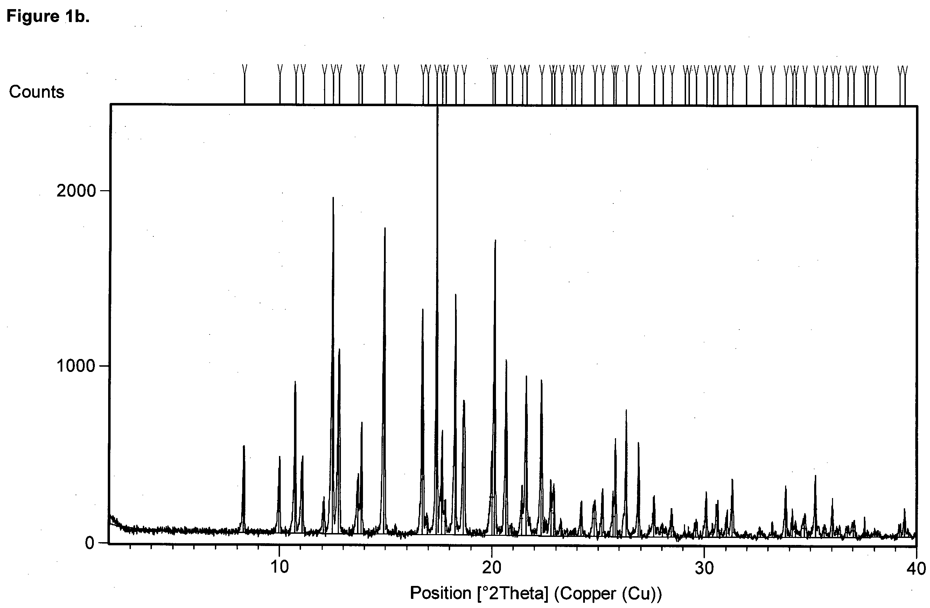

**Figure 1b****.** X-ray powder record of the scopine ester **I,** form A (the form described in patent EP0418716)

### Examples

The melting temperatures were measured using a Kofler block.

X-ray powder diffraction measurement parameters: The diffraction patterns were measured using an X'PERT PRO MPD PANalytical diffractometer with a graphite monochromator, radiation used: CuKα (λ=1.542 A), excitation voltage: 45 kV, anode current: 40 mA, measured range: 2 - 40° 2θ, increment: 0.01° 2θ. The measurement was carried out using a flat powder sample that was placed on a Si plate. For the primary optic setting programmable divergence diaphragms with the irradiated sample area of 10 mm, Soller diaphragms 0.02 rad and an anti-dispersion diaphragm % were used. For the secondary optic setting an X'Celerator detector with the maximum opening of the detection slot, Soller diaphragms 0.02 rad and an anti-dispersion diaphragm 5.0 mm were used

### Method of UPLC analysis of the scopine ester of formula I

### Mobile phase:

A: Potassium hydrogen phosphate 0.01 M, pH 7.8 ± 0.05
B: acetonitrile R
Column: UPLC BEH Shield RP 18 or equivalent
Elution: gradient

| **Time (min)** | **% A** | **% B** |
|---|---|---|
| 0.0 | 80 | 20 |
| 5.0 | 30 | 70 |
| 7.0 | 30 | 70 |
| 7.5 | 80 | 20 |

Flow rate: 0.4 ml/min.
Column temperature 15°C
Autosampler temperature: 20°C
Injection: 1 µl
Analysis time: 7.5 min
Equilibration time: 2.5 min
Detection: 238 nm

### Sample preparation:

25.0 mg of the tested substance is dissolved in the solvent acetonitrile - water (1+1). It is put in an ultrasonic bath for 1 min and, after cooling to the laboratory temperature, diluted with the same solvent to 25.0 ml.

### Preparation of the scopine ester of formula I

### Example 1

A dissolved mixture of scopine (500g, 3.22 mmol) a triethylamine (450 µl, 1 equiv.) in 4ml of tetrahydrofuran was gradually added dropwise to a stirred solution of oxalyl chloride (286 µl, 1.05 equiv.) and dimethylaminopyridine (19.7 mg, 0.05 equiv.) in tetrahydrofuran (4 mL) at the temperature of 0°C at the rate of 15 ml/h. The reaction mixture was stirred at 0°C for 2 hours. Subsequently, the mixture was cooled to -30°C and, during 1 h, a solution of 2-thienylmagnesium bromide (3 equivalents, 9.66ml of 1M solution in tetrahydrofuran) was added dropwise and then the reaction mixture was stirred at -30°C for another 2 h. The solution was then diluted with toluene (30 ml) and admixed to a mixture of 40 g of ice and 10 ml of 2M HCl. The acidic phase is separated, alkalized with 2M Na₂CO₃ to pH 9 and the free base of the scopine ester I is extracted with dichloromethane (4x 20ml). After re-drying with Na₂SO₄ dichloromethane is evaporated at a reduced pressure. The obtained crude product (0.750 g) with the purity of 82.98% (analyzed with UPLC) was crystallized from acetonitrile by cooling of the solution to the temperature of -32°C for 80 h. The crystallized product was filtered, washed with a minimum quantity of cooled acetonitrile and dried freely at the room temperature. 0.323 g of the scopine ester of formula **I,** form B of the purity of 98.28% (analyzed with UPLC) was obtained; melting point 117.4-118.0 °C in the yield of 31%. The X-ray record is presented in the Annex in fig. **1a****.**

**X-ray powder diffraction - Diffraction peaks of the scopine ester of formula I, form B**

| Pos. [°2Th.] | d-spacing [A] | Rel. Int. [%] |
|---|---|---|
| 8.24 | 10.722 | 7.0 |
| **10.03** | 8.812 | 100.0 |
| **12.00** | 7.367 | 25.4 |
| 13.09 | 6.757 | 11.6 |
| **14.03** | 6.307 | 20.6 |
| **16.53** | 5.358 | 18.9 |
| 18.13 | 4.889 | 13.5 |
| 18.94 | 4.682 | 5.3 |
| **20.10** | 4.415 | 38.5 |
| 23.18 | 3.834 | 6.3 |
| 24.27 | 3.665 | 3.2 |
| 25.03 | 3.554 | 2.5 |
| **26.24** | 3.394 | 6.2 |
| 26.92 | 3.310 | 3.6 |
| 33.43 | 2.678 | 6.1 |

### Example 2

A dissolved mixture of scopine (1 g, 6.44 mmol) and triethylamine (900 µl, 1 equiv.) in 8 ml of tetrahydrofuran was slowly (at the rate of 15 ml/h) added dropwise to a stirred solution of oxalyl chloride (572 µl, 1.05 equivalents) and dimethylaminopyridine (39.4 mg, 0.05 equiv) in tetrahydrofuran (8 ml) at 0°C. The reaction mixture was stirred at 0°C for 2 hours. Then the mixture was cooled to -30°C and, during 1 hour, a solution of 2-thienylmagnesium bromide (3 equivalents, 19.32ml of 1 M solution in tetrahydrofuran) was added dropwise (at the rate of 37 ml/h), then the reaction mixture was stirred at -30°C for another 2 hours. The solution is then diluted with toluene (60 ml) and admixed to a mixture of 80 g of ice and 20 ml of 2M HCl. The acidic phase is separated, alkalized with 2M Na₂CO₃ to pH 9 and the free base of the scopine ester **I** is extracted with dichloromethane (3x 100 ml). After re-drying with Na₂SO₄ dichloromethane is evaporated at a reduced pressure. The crude mixture was dissolved in acetone and filtered on a low column of silica gel. The obtained crude product (0.939 g) was crystallized from acetonitrile (8 ml) by cooling the solution to -32°C. The crystallized product was filtered, washed with a minimum amount of cooled acetonitrile and dried in a vacuum drier at 45°C for five hours. 0.564 g of the scopine ester **I,** form A was obtained with the purity of 97.69% (analyzed with UPLC), melting point 148.7-150.0°C in the yield of 25%. The X-ray record is presented in the Annex in fig. **1** **b.**

**X-ray powder diffraction - Diffraction peaks of the scopine ester of formula I, form A**

| Pos. [°2Th.] | d-spacing [A] | Rel. Int. [%] |
|---|---|---|
| 8.31 | 10.635 | 20.4 |
| 9.98 | 8.855 | 15.2 |
| **10.72** | 8.245 | 35.8 |
| 11.06 | 7.994 | 17.4 |
| **12.48** | 7.090 | 76.6 |
| 12.78 | 6.923 | 41.5 |
| 13.85 | 6.390 | 22.9 |
| **14.91** | 5.938 | 68.2 |
| 16.71 | 5.302 | 53.7 |
| **17.38** | 5.099 | 100.0 |
| 17.63 | 5.026 | 20.4 |
| 18.25 | 4.856 | 55.8 |
| 18.66 | 4.752 | 31.9 |
| **20.12** | 4.411 | 60.3 |
| 20.65 | 4.297 | 36.7 |
| 21.59 | 4.113 | 35.3 |
| **22.31** | 3.981 | 37.0 |
| 22.76 | 3.903 | 12.0 |
| 24.81 | 3.586 | 8.2 |
| 25.19 | 3.532 | 10.4 |
| 25.81 | 3.450 | 21.8 |
| **26.30** | 3.386 | 26.3 |
| 26.89 | 3.313 | 21.0 |
| 27.61 | 3.228 | 9.2 |
| 30.08 | 2.968 | 9.5 |
| **31.31** | 2.855 | 13.1 |
| 33.83 | 2.648 | 10.9 |

## Claims

1. A method of preparing the scopine ester of formula I, 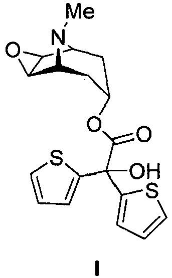 **characterized in that** it consists of the following steps:
a) reaction of scopine of formula III with derivatives of oxalic acid of formula XIII 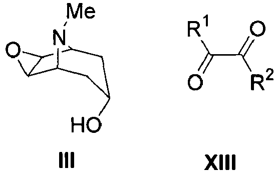 wherein X means F, Cl, Br, or I, and R is X or *O*-*tert*-butyl, *O*-methyl, *N-*pyrrolidinyl, *N*-morpholinyl or *N*-imidazolyl, in the presence of a weak base and a catalyst in an inert organic solvent, producing a derivative of formula XIV 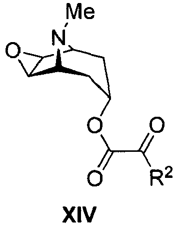
b) reaction of the derivative of formula XIV with at least 2 equivalents of 2-thienylmagnesium bromide of formula XV 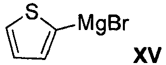
c) isolation of the resulting scopine ester of formula I and crystallization from a crystallization solvent.

2. The method in accordance with claim 1, **characterized in that** the compound of formula XIV is not isolated in step a).

3. The method in accordance with claims 1 or 2, **characterized in that** each of R¹ and R² in the compound of formula XIII is chlorine.

4. The method in accordance with claims 1 or 2, **characterized in that** the selected weak base in step a) is a tertiary amine.

5. The method in accordance with claims 1 or 2, **characterized in that** the selected catalyst in step a) is dimethylaminopyridine.

6. The method in accordance with claims 1 or 2, **characterized in that** the solvent in step a) is selected from aliphatic or cyclic ethers.

7. The method in accordance with claim 6, **characterized in that** the selected solvent is tetrahydrofuran.

8. The method in accordance with claims 1 or 2, **characterized in that** the amount of 2-thienylmagnesium bromide of formula XV in step b) is 2 to 3.5 equivalents.

9. The method in accordance with claims 1 or 2, **characterized in that** the isolation is carried out by means of chromatography.

10. The method in accordance with claim 9, **characterized in that** the isolated product is crystallized from toluene or acetonitrile, or their mixtures.

## Patentansprüche

1. Verfahren zur Herstellung des Scopinesters der Formel I, 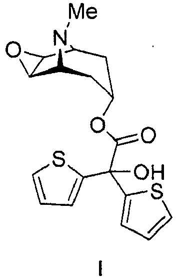 **dadurch gekennzeichnet, dass** es aus den folgenden Schritten besteht:
a) Umsetzung von Scopin der Formel III mit Derivaten der Oxalsäure der Formel XIII 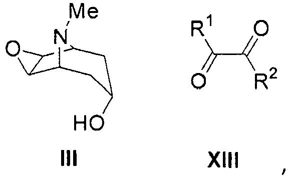 in der X F, Cl, Br oder I ist und R X oder O-*tert*-Butyl, O-Methyl, N-Pyrrolidinyl, N-Morpholinyl oder N-Imidazolyl ist, in Gegenwart einer schwachen Base und eines Katalysators in einem inerten organischen Lösungsmittel, wodurch ein Derivat der Formel XIV erhalten wird 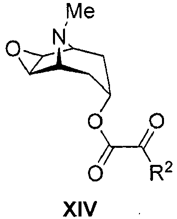
b) Umsetzung des Derivats der Formel XIV mit mindestens 2 Äquivalenten von 2-Thienylmagnesiumbromid der Formel XV 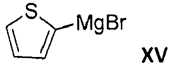
c) Isolieren des resultierenden Scopinesters der Formel I und Kristallisieren aus einem Kristallisationslösungsmittel.

2. Verfahren nach Anspruch 1, **dadurch gekennzeichnet, dass** die Verbindung der Formel XIV nicht in Schritt a) isoliert wird.

3. Verfahren nach Anspruch 1 oder 2, **dadurch gekennzeichnet, dass** R¹ und R² in der Verbindung der Formel XIII jeweils Chlor sind.

4. Verfahren gemäß Anspruch 1 oder 2, **dadurch gekennzeichnet, dass** die gewählte schwache Base in Schritt a) ein tertiäres Amin ist.

5. Verfahren gemäß Anspruch 1 oder 2, **dadurch gekennzeichnet, dass** der gewählte Katalysator in Schritt a) Dimethylaminopyridin ist.

6. Verfahren gemäß Anspruch 1 oder 2, **dadurch gekennzeichnet, dass** das Lösungsmittel in Schritt a) aus aliphatischen oder cyclischen Ethern ausgewählt ist.

7. Verfahren nach Anspruch 6, **dadurch gekennzeichnet, dass** das gewählte Lösungsmittel Tetrahydrofuran ist.

8. Verfahren nach Anspruch 1 oder 2, **dadurch gekennzeichnet, dass** die Menge an 2-Thienylmagnesiumbromid der Formel XV in Schritt b) 2 bis 3,5 Äquivalente beträgt.

9. Verfahren gemäß Anspruch 1 oder 2, **dadurch gekennzeichnet, dass** das Isolieren mittels Chromatographie erfolgt.

10. Das Verfahren nach Anspruch 9, **dadurch gekennzeichnet, dass** das isolierte Produkt aus Toluol oder Acetonitril, oder aus deren Mischungen, kristallisiert wird.

## Revendications

1. Un procédé de préparation d'ester de scopine de formule I, 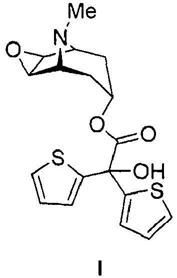 **caractérisé en ce qu'**il consiste en les étapes suivantes:
a) réaction de scopine de formule III avec des dérivés d'acide oxalique de formule XIII 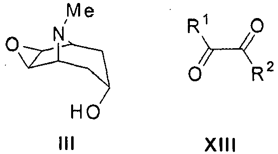 formule dans laquelle
▪ X est F, Cl, Br, ou I, et
▪ R est X ou un groupe *O*-*tert*-butyl, *O*-méthyl, *N*-pyrrolidinyl, *N*-morpholinyl ou *N*-imidazolyl,
en présence d'une base faible et d'un catalyseur dans un solvant organique inerte, pour produire un dérivé de formule XIV 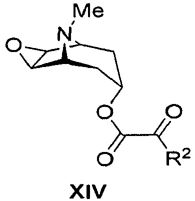
b) réaction du dérivé de formule XIV avec au moins 2 équivalents de bromure de 2-thiénylmagnésium de formule XV 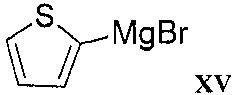
c) isolement de l'éther de scopine de formule I résultant et cristallisation dans le solvant de cristallisation.

2. Le procédé selon la revendication 1, **caractérisé en ce que** le composé de formule XIV n'est pas isolé dans l'étape a).

3. Le procédé selon les revendications 1 ou 2, **caractérisé en ce que** chacun des R¹ et R² dans le composé de formule XIII est du chlore.

4. Le procédé selon les revendications 1 ou 2, **caractérisé en ce que** la base faible sélectionnée pour l'étape a) est une amine tertiaire.

5. Le procédé selon les revendications 1 ou 2, **caractérisé en ce que** le catalyseur sélectionné pour l'étape a) est la diméthylaminopyridine.

6. Le procédé selon les revendications 1 ou 2, **caractérisé en ce que** le solvant dans l'étape a) est sélectionné parmi les éthers aliphatiques ou les éthers cycliques.

7. Le procédé selon les revendications 6, **caractérisé en ce que** le solvant sélectionné est le tétrahydrofuranne.

8. Le procédé selon les revendications 1 ou 2, **caractérisé en ce que** la quantité de bromure de 2-thiénylmagnésium de formule XV dans l'étape b) est de 2 à 3,5 équivalents.

9. Le procédé selon les revendications 1 ou 2, **caractérisé en ce que** l'isolement est mis en oeuvre par chromatographie.

10. Le procédé selon les revendications 9, **caractérisé en ce que** le produit isolé est cristallisé dans le toluène ou dans l'acétonitrile, ou de leurs mélanges.
